# EUROPEAN PATENT APPLICATION

(11) **EP 4 564 232 A1**
(43) Date of publication of application: **04.06.2025**
(21) Application number: 22952608.2
(22) Date of filing: 10.08.2022
(51) Int. Cl.: G06N 3/12, C12M 1/12, C12M 1/40

(54) **WRITING SYSTEM AND WRITING METHOD FOR MOLECULAR DATA STORAGE, AND WRITING CONTROL DEVICE**

(30) Priority: 29.07.2022 CN 202210905754
(71) Applicant: Digicodon Technologies Co., Ltd., Hangzhou, Zhejiang 311121 (CN)
(72) Inventor: ZHANG, Lushuai, Hangzhou, Zhejiang 311121 (CN); JIANG, Shuo, Hangzhou, Zhejiang 311121 (CN)
(74) Representative: Torggler & Hofmann Patentanwälte - Innsbruck
(86) International application number: PCT/CN2022/111487
(87) International publication number: WO 2024/021164

(57) **Abstract**

Present disclosure is related to a writing system for molecular data storage, comprising: an encoded data parsing unit configured to acquire encoded molecular module assembly data and parse the molecular module assembly data into writing process control instructions; a storage unit configured to store predetermined types of molecular modules and provide corresponding molecular modules according to the writing process control instructions; a dispensing unit configured to acquire corresponding molecular modules from the storage unit and dispense the corresponding molecular modules to predetermined reaction sites on a substrate according to the writing process control instructions, wherein each of the predetermined reaction sites is provided with molecular modules required for one or more predetermined reaction processes; and a reaction unit configured to facilitate one or more predetermined reaction processes at each of the predetermined reaction sites on the substrate respectively, thereby generating one or more predetermined compositions at each of the predetermined reaction sites on the substrate respectively. Further, present disclosure is also related to a writing method and a writing control device for molecular data storage (Fig. 1).

## Description

### TECHNICAL FIELD

The present disclosure relates to a field of molecular storage technology, in particular, to a writing system, a writing method and a writing control device for molecular data storage, such as DNA data storage.

### BACKGROUND

With the substantial development of information technology, people's demand for data storage is increasing rapidly. Traditional data storage media include hard disks, flash memories, magnetic tapes, optical disks, etc., which have problems such as low storage density, short storage time, and high energy consumption. In order to achieve higher storage density and more reliable storage effect, a method and a device related to storing information in a molecule have been proposed at present. Taking data storage using a DNA molecule as an example, its storage density can theoretically reach more than 106 to 107 times that of the traditional storage media, reducing the cost of data storage operation and maintenance by orders of magnitude. In addition, the DNA is very stable, and data therein can be preserved for more than a thousand years under dry and low-temperature conditions. In addition, the DNA storage also has great advantages over traditional storage manners in terms of carbon emissions and energy consumption, data security, portability, and the like.

However, there is currently a lack of suitable writing systems and methods for molecular data storage, which results in unsatisfactory performance in terms of writing quality, writing speed, and/or writing cost. Therefore, there is a demand for improvements in the existing writing technologies for molecular data storage.

### SUMMARY

One of the objects of the present disclosure is to provide an improved writing system, writing method, and writing control device in the field of writing technology for molecular data storage.

According to a first aspect of the present disclosure, there is provided a writing system for molecular data storage, characterized in that the writing system comprises:
an encoded data parsing unit configured to acquire encoded molecular module assembly data and parse the molecular module assembly data into writing process control instructions;
a storage unit configured to store predetermined types of molecular modules and provide corresponding molecular modules according to the writing process control instructions;
a dispensing unit configured to acquire corresponding molecular modules from the storage unit and dispense the corresponding molecular modules to predetermined reaction sites on a substrate according to the writing process control instructions, wherein each of the predetermined reaction sites is provided with molecular modules required for one or more predetermined reaction processes; and
a reaction unit configured to facilitate one or more predetermined reaction processes at each of the predetermined reaction sites on the substrate respectively, thereby generating one or more predetermined compositions at each of the predetermined reaction sites on the substrate respectively.

In some embodiments, the encoded data parsing unit comprises an encoding module configured to acquire initial information for molecular data storage and encode the initial information into the molecular module assembly data.

In some embodiments, the molecular module assembly data comprises position encoded data and content encoded data associated with the position encoded data.

In some embodiments, the molecular module assembly data is configured as degeneracy-calculated molecular module assembly data, such that the respective content encoded data for at least a portion of the predetermined reaction sites involves molecular modules required for multiple predetermined reaction processes, thereby generating corresponding multiple predetermined compositions at the at least a portion of the predetermined reaction sites on the substrate respectively.

In some embodiments, the encoded data parsing unit comprises a translation module configured to acquire molecular module assembly data and convert the molecular module assembly data into writing process control instructions, wherein the writing process control instructions comprise molecular module scheduling instructions for the storage unit and molecular module dispensing instructions for the dispensing unit.

In some embodiments, the storage unit comprises multiple storage containers for various predetermined molecular modules, wherein a solution and molecular modules dissolved in the solution are stored in the respective storage container, and/or the storage unit comprises storage containers for storing enzymes involved in the reaction process.

In some embodiments, additives are stored in the corresponding storage containers, and the additives are configured to promote the reaction process in which the corresponding molecular modules participate and/or facilitate the dispensing process of the corresponding molecular modules by the dispensing unit.

In some embodiments, the additives include salts, glycerol, polyethylene glycol, and/or surfactants.

In some embodiments, the storage unit comprises a molecular module supply device, which connects the corresponding storage containers to the dispensing unit via multiple supply pipelines, and delivers the corresponding molecular modules and/or enzymes to the dispensing unit through the corresponding supply pipelines according to the writing process control instructions.

In some embodiments, the storage unit comprises a molecular module preparation device, which is configured to synthesize predetermined molecular modules and/or replicate predetermined molecular modules.

In some embodiments, the dispensing unit comprises a print head and a writing control device for controlling the printing process of the print head, wherein the writing control device is configured to control the relative displacement between the print head and the substrate according to the writing process control instructions, so that the print head dispenses reaction droplets containing corresponding molecular modules to the predetermined reaction sites on the substrate in accordance with the molecular module assembly data.

In some embodiments, the print head is configured to dispense reaction droplets containing corresponding molecular modules to the predetermined reaction sites on the substrate in a scanning printing or single-pass printing manner.

In some embodiments, the dispensing unit comprises multiple print heads, and the multiple print heads are arranged in: a single-column arrangement parallel to the movement direction of the substrate; a single-row arrangement parallel to the movement direction of the print heads; a matrix arrangement; or an irregular arrangement.

In some embodiments, each print head comprises multiple nozzles configured to dispense reaction droplets containing predetermined molecular modules at nanoliter or picoliter level to the predetermined reaction sites on the substrate.

In some embodiments, the writing control device is configured to control the printing process of the print head according to the writing process control instructions, such that molecular modules required for a first number of predetermined reaction processes are dispensed to a first portion of the predetermined reaction sites on the substrate respectively, thereby generating corresponding first number of predetermined compositions at the first portion of the predetermined reaction sites respectively, and molecular modules required for a second number of predetermined reaction processes are dispensed to a second portion of the predetermined reaction sites on the substrate respectively, thereby generating corresponding second number of predetermined compositions at the second portion of the predetermined reaction sites respectively, wherein the first number is greater than the second number.

In some embodiments, the writing control device is configured to control the printing process of the print head according to the writing process control instructions, such that molecular modules required for multiple predetermined reaction processes are dispensed to a first portion of predetermined reaction sites on the substrate respectively, thereby generating corresponding multiple predetermined compositions at the first portion of predetermined reaction sites respectively, and molecular modules required for only one predetermined reaction process are dispensed to a second portion of predetermined reaction sites on the substrate respectively, thereby generating corresponding only one predetermined composition at the second portion of predetermined reaction sites respectively.

In some embodiments, each of the first portion of the predetermined reaction sites occupies a larger area on the substrate compared to each of the second portion of the predetermined reaction sites.

In some embodiments, each of the first portion of the predetermined reaction sites is dispensed more types of molecular modules compared to each of the second portion of the predetermined reaction sites.

In some embodiments, each of the first portion of the predetermined reaction sites is dispensed reaction droplets of larger volume compared to each of the second portion of the predetermined reaction sites.

In some embodiments, the print head comprises a piezoelectric print head, a thermal bubble print head, or a solenoid valve print head.

In some embodiments, the substrate is configured as a continuous substrate and transported in a roll-to-roll manner.

In some embodiments, the substrate is configured as a discrete substrate and transported in a sheet-to-sheet manner.

In some embodiments, the dispensing unit comprises a static electricity eliminator for eliminating static electricity from the substrate.

In some embodiments, the static electricity eliminator is configured as an ion flow electrostatic eliminator, a radioactive isotope electrostatic eliminator, or a sparkless electrostatic eliminator.

In some embodiments, the dispensing unit comprises a cleaning liquid chamber and cleaning pipelines, wherein the cleaning liquid chamber stores cleaning liquid for cleaning the print head and the associated cleaning pipelines.

In some embodiments, the dispensing unit comprises a dispensing detection module configured to detect the accuracy of the dispensing of reaction droplets by the print head.

In some embodiments, the dispensing detection module comprises a microscope device and/or an image acquisition device.

In some embodiments, the image acquisition device is configured to acquire a first detection image of at least a part of the substrate.

In some embodiments, the dispensing unit comprises a test area, and the writing control device is configured to control the printing process of the print head according to a test instruction, so that the print head dispenses reaction droplets containing predetermined molecular modules to predetermined test sites on the test area.

In some embodiments, the image acquisition device is configured to acquire a second detection image of the test area.

In some embodiments, the dispensing detection module comprises an analysis device, wherein a first expected image related to the at least a part of the substrate and/or a second expected image related to the test area is stored in the analysis device, and the analysis device is configured to compare the first detection image with the first expected image and/or the second detection image with the second expected image.

In some embodiments, the analysis device is configured to compare the first detection image with the first expected image and/or the second detection image with the second expected image in terms of the positional distribution of reaction droplets, the size of reaction droplets, and/or the color of reaction droplets in the image.

In some embodiments, the analysis device is configured to generate feedback data to reflect the discrepancy when a discrepancy is identified between the first detection image and the first expected image and/or the second detection image and the second expected image.

In some embodiments, the feedback data is configured as a feedback table, wherein the feedback table presents specific discrepancies and the positions of reaction droplets related to the discrepancies.

In some embodiments, the writing control device is configured to control the printing process of the print head based on the feedback data, so that the print head performs supplementary printing on the substrate.

In some embodiments, the dispensing unit comprises a pre-processing module configured to pre-process the substrate.

In some embodiments, the pre-processing module is configured to perform hydrophilic treatment on the predetermined reaction sites of the substrate and/or hydrophobic treatment on areas other than the predetermined reaction sites of the substrate.

In some embodiments, the reaction unit comprises an enzyme addition device for adding enzymes to the predetermined reaction sites on the substrate.

In some embodiments, the reaction unit comprises a physical oscillation module configured to optimize the mixing of reaction droplets at the respective predetermined reaction sites on the substrate.

In some embodiments, the reaction unit comprises an oil droplet addition device for adding oil droplets to the predetermined reaction sites on the substrate, so that the oil droplets surround the reaction droplets respectively, thereby reducing the evaporation of the reaction droplets.

In some embodiments, the reaction unit comprises a constant temperature and humidity control chamber, wherein multiple rollers are provided in the control chamber for the substrate to pass through in a rotating manner.

In some embodiments, the writing system comprises a reaction termination unit for terminating the reaction at the predetermined reaction sites on the substrate.

In some embodiments, the reaction termination unit comprises: a temperature adjustment device and/or a humidity adjustment device configured to deactivate enzymes; and/or a termination chemical addition device configured to add termination chemicals to the substrate.

In some embodiments, the termination chemicals include ethylenediaminetetraacetic acid (EDTA) and/or salts above a predetermined concentration.

In some embodiments, the writing system comprises a reaction droplet elution unit, which is configured to elute reaction droplets containing compositions from the substrate into an aqueous or oil phase medium.

In some embodiments, the writing system comprises a reaction droplet collection unit, which is configured to receive reaction droplets containing compositions eluted by the reaction droplet elution unit.

In some embodiments, the reaction droplet collection unit comprises a composition processing module, which is configured to: screen effective compositions from the eluted reaction droplets; and back up the effective compositions through a predetermined amplification method.

In some embodiments, the composition processing module is configured to: perform enrichment and collection processing on the eluted reaction droplets before screening.

In some embodiments, the enrichment and collection processing is performed as follows: removing the aqueous phase from the eluted reaction droplets by column or membrane filtration; and collecting effective compositions by elution, magnetic bead methods, freeze-drying, and/or heat drying.

In some embodiments, the writing system comprises a molecular data transfer module, which is configured to transfer the collection of effective compositions to a molecular data storage device.

In some embodiments, the molecular modules include at least one of deoxyribonucleic acid (DNA), ribonucleic acid (RNA), peptides, organic polymers, and molecular fragments arranged at intervals.

In some embodiments, the molecular modules are distinguished by at least one of their sequence distribution, sequence length, and secondary structure.

In some embodiments, the content encoded data of the molecular module assembly data comprises content information encoded data and feature information encoded data associated with the content information.

In some embodiments, the encoded data parsing unit is configured to acquire molecular module assembly data in the form of a first matrix, wherein assembly information associated with the predetermined reaction sites on the substrate is stored in the first matrix.

In some embodiments, the encoded data parsing unit is configured to convert the first matrix into a second matrix, wherein operation instructions for molecular module dispensing are stored in the second matrix.

In some embodiments, the encoded data parsing unit is configured to convert the first matrix into multiple second matrices associated with multiple print heads in the dispensing unit.

In some embodiments, operation instructions for the corresponding print head are stored in each second matrix.

In some embodiments, the encoded data parsing unit is configured to combine the operation instructions stored in the second matrices into writing process control instructions.

According to a second aspect of the present disclosure, there is provided a writing method for molecular data storage, comprising:
acquiring writing process control instructions converted from encoded molecular module assembly data, wherein the molecular module assembly data comprises position encoded data and content encoded data associated with the position encoded data;
dispensing corresponding molecular modules to predetermined reaction sites on a substrate according to the writing process control instructions, wherein each of the predetermined reaction sites is provided with molecular modules required for one or more predetermined reaction processes respectively, thereby generating one or more predetermined compositions at each of the predetermined reaction sites on the substrate respectively.

In some embodiments, the molecular module assembly data is configured as degeneracy-calculated molecular module assembly data, such that the content encoded data for at least a portion of the predetermined reaction sites involves molecular modules required for multiple predetermined reaction processes respectively, thereby generating corresponding multiple predetermined compositions at the at least a portion of the predetermined reaction sites on the substrate respectively.

In some embodiments, "dispensing corresponding molecular modules to predetermined reaction sites on the substrate according to the writing process control instructions" comprises: controlling the relative displacement between the print head and the substrate according to the writing process control instructions, so that the print head dispenses reaction droplets containing the corresponding molecular modules to the predetermined reaction sites on the substrate in a scanning printing or single-pass printing manner in accordance with the molecular module assembly data.

In some embodiments, "dispensing corresponding molecular modules to predetermined reaction sites on a substrate according to the writing process control instructions" comprises: controlling the printing process of the print head according to the writing process control instructions, so that molecular modules required for a first number of predetermined reaction processes are dispensed to a first portion of the predetermined reaction sites on the substrate respectively, thereby generating corresponding first number of predetermined compositions at the first portion of the predetermined reaction sites respectively, wherein the first number is greater than or equal to two, and/or controlling the printing process of the print head according to the writing process control instructions, so that molecular modules required for a second number of predetermined reaction processes are dispensed to a second portion of the predetermined reaction sites on the substrate respectively, thereby generating corresponding second number of predetermined compositions at the second portion of the predetermined reaction sites respectively, wherein the second number is less than the first number.

In some embodiments, "dispensing corresponding molecular modules to predetermined reaction sites on the substrate according to the writing process control instructions" comprises: controlling the printing process of the print head according to the writing process control instructions, such that molecular modules required for multiple predetermined reaction processes are dispensed to a first portion of predetermined reaction sites on the substrate respectively, thereby generating corresponding multiple predetermined compositions at the first portion of predetermined reaction sites respectively, and/or controlling the printing process of the print head according to the writing process control instructions, such that molecular modules required for only one predetermined reaction process are dispensed to a second portion of predetermined reaction sites on the substrate respectively, thereby generating corresponding only one predetermined composition at the second portion of predetermined reaction sites respectively.

In some embodiments, compared to each of the second portion of the predetermined reaction sites: each of the first portion of the predetermined reaction sites occupies a larger area on the substrate; each of the first portion of the predetermined reaction sites is dispensed more types of molecular modules; and/or each of the first portion of the predetermined reaction sites is dispensed reaction droplets of larger volume.

In some embodiments, the writing method comprises: performing static electricity elimination treatment on the substrate by means of a static electricity eliminator.

In some embodiments, the static electricity eliminator is configured as an ion flow electrostatic eliminator, a radioactive isotope electrostatic eliminator, or a sparkless electrostatic eliminator.

In some embodiments, the writing method comprises detecting the accuracy of the dispensing of reaction droplets by a dispensing detection module.

In some embodiments, "detecting the accuracy of the dispensing of reaction droplets by a dispensing detection module" comprises: acquiring a first detection image of at least a part of the substrate and comparing the first detection image with a first expected image of the at least a part of the substrate; and/or acquiring a second detection image of a test area and comparing the second detection image with a second expected image of the test area.

In some embodiments, the writing method comprises comparing the first detection image with the first expected image and/or the second detection image with the second expected image in terms of the positional distribution of reaction droplets, the size of reaction droplets, and/or the color of reaction droplets in the image.

In some embodiments, the writing method comprises generating feedback data to reflect the discrepancy when the discrepancy is identified between the first detection image and the first expected image and/or the second detection image and the second expected image.

In some embodiments, the feedback data is configured as a feedback table, wherein the feedback table presents specific discrepancies and the positions of reaction droplets related to the discrepancies.

In some embodiments, the writing method comprises supplementarily dispensing molecular modules to the substrate based on the feedback data.

In some embodiments, the writing method comprises: preprocessing the substrate according to the molecular module assembly data, wherein the preprocessing comprises performing hydrophilic treatment on the predetermined reaction sites of the substrate and/or performing hydrophobic treatment on areas of the substrate other than the predetermined reaction sites.

In some embodiments, the writing method comprises: dispensing corresponding enzymes to the predetermined reaction sites on the substrate according to the writing process control instructions.

In some embodiments, the writing method comprises: acquiring initial information for molecular data storage and encoding the initial information into the molecular module assembly data.

In some embodiments, the writing method comprises: converting the molecular module assembly data into writing process control instructions by means of a translation module, wherein the writing process control instructions include molecular module scheduling instructions and molecular module dispensing instructions.

In some embodiments, the writing method comprises: optimizing the mixing of reaction droplets at the predetermined reaction sites on the substrate by means of a physical oscillation module.

In some embodiments, the writing method comprises: adding oil droplets to the predetermined reaction sites on the substrate, so that the oil droplets surround the reaction droplets, thereby reducing the evaporation of the reaction droplets.

In some embodiments, the writing method comprises: terminating the reaction at the predetermined reaction sites on the substrate by means of a reaction termination unit, wherein the reaction termination unit comprises: a temperature adjustment device and/or a humidity adjustment device configured to deactivate enzymes; and/or a termination chemical addition device configured to add termination chemicals to the substrate.

In some embodiments, the writing method comprises: eluting reaction droplets containing compositions from the substrate into an aqueous or oil phase medium by means of a reaction droplet elution unit.

In some embodiments, the writing method comprises: receiving reaction droplets eluted by the reaction droplet elution unit containing compositions by means of a reaction droplet collection unit.

In some embodiments, the writing method comprises: screening effective compositions from the eluted reaction droplets; and backing up the effective compositions through a predetermined amplification method.

In some embodiments, the writing method comprises: performing enrichment and collection processing on the eluted reaction droplets before screening, wherein the enrichment and collection processing is performed as follows: removing the aqueous phase from the eluted reaction droplets by column or membrane filtration; and collecting effective compositions by elution, magnetic bead methods, freeze-drying, and/or heat drying.

In some embodiments, the writing method comprises: transferring the collection of effective compositions to a molecular data storage device by means of a molecular data transfer module.

In some embodiments, the molecular modules include at least one of deoxyribonucleic acid (DNA), ribonucleic acid (RNA), peptides, organic polymers, and molecular fragments arranged at intervals.

In some embodiments, the writing method comprises: distinguishing various molecular modules by at least one of their sequence distribution and sequence length.

In some embodiments, the content encoded data of the molecular module assembly data comprises content information encoded data and feature information encoded data associated with the content information.

In some embodiments, the writing method comprises: acquiring molecular module assembly data in the form of a first matrix, wherein assembly information associated with the predetermined reaction sites on the substrate is stored in the first matrix.

In some embodiments, the writing method comprises: converting the first matrix into a second matrix, wherein operation instructions for molecular module dispensing are stored in the second matrix.

In some embodiments, the writing method comprises: converting the first matrix into multiple second matrices associated with multiple print heads in the dispensing unit.

In some embodiments, operation instructions for the corresponding print head are stored in each second matrix.

In some embodiments, the writing method comprises: combining the operation instructions stored in the second matrices into writing process control instructions.

According to a third aspect of the present disclosure, there is provided a writing control device for molecular data storage, comprising: at least one memory storing writing process control instructions; and at least one processor configured to execute the writing process control instructions, wherein the at least one processor implements the method according to some embodiments of present disclosure when executing the writing process control instructions.

According to a fourth aspect of the present disclosure, there is provided an encoded data parsing unit for molecular data storage, characterized in that the encoded data parsing unit comprises:
a molecular data encoding system configured to acquire initial information to be stored and convert the initial information to be stored into encoded molecular module assembly data, wherein the molecular module assembly data comprises position encoded data and content encoded data associated with the position encoded data;
a molecular data code translation system configured to acquire the encoded molecular module assembly data and parse the molecular module assembly data into writing process control instructions,
wherein the molecular data code translation system is configured to: acquire a first matrix formed by various molecular module assembly data, wherein assembly information associated with the predetermined reaction sites on the substrate is stored in the first matrix; convert the first matrix into a second matrix, wherein operation instructions for molecular module dispensing are stored in the second matrix; combine the operation instructions stored in the second matrix into writing process control instructions.

In some embodiments, the molecular data code translation system is configured to: convert the first matrix into multiple second matrices associated with multiple print heads in the dispensing unit, wherein operation instructions for the corresponding print head are stored in each second matrix.

In some embodiments, the molecular data encoding system is configured to configure the content encoded data of the molecular module assembly data to include content information encoded data and feature information encoded data associated with the content information.

According to a fifth aspect of the present disclosure, there is provided a dispensing unit for molecular data storage, characterized in that the dispensing unit comprises: a molecular module dispensing system comprising at least one print head; a molecular module dispensing control system comprising the writing control device according to present disclosure, wherein the writing control device is configured to control the printing process of the print head of the molecular module dispensing system on the substrate.

In some embodiments, the dispensing unit includes a static electricity eliminator for eliminating static electricity from the substrate.

In some embodiments, the static electricity eliminator is configured as an ion flow electrostatic eliminator, a radioactive isotope electrostatic eliminator, or a sparkless electrostatic eliminator.

In some embodiments, the dispensing unit includes a cleaning liquid chamber and cleaning pipelines, wherein the cleaning liquid chamber stores cleaning liquid for cleaning the print head and the associated cleaning pipelines.

In some embodiments, the dispensing unit includes a dispensing detection module, which is configured to detect the accuracy of the dispensing of reaction droplets by the print head.

In some embodiments, the dispensing detection module includes a microscope device and/or an image acquisition device.

In some embodiments, the image acquisition device is configured to acquire a first detection image of at least a part of the substrate.

In some embodiments, the dispensing unit includes a test area, and the writing control device is configured to control the printing process of the print head according to a test instruction, so that the print head dispenses reaction droplets containing predetermined molecular modules to predetermined test sites in the test area.

In some embodiments, the image acquisition device is configured to acquire a second detection image of the test area.

In some embodiments, the dispensing detection module includes an analysis device, wherein a first expected image related to the at least a part of the substrate and/or a second expected image related to the test area is stored in the analysis device, and the analysis device is configured to compare the first detection image with the first expected image and/or the second detection image with the second expected image.

In some embodiments, the analysis device is configured to compare the first detection image with the first expected image and/or the second detection image with the second expected image in terms of the positional distribution of reaction droplets, the size of reaction droplets, and/or the color of reaction droplets in the image.

In some embodiments, the analysis device is configured to generate feedback data to reflect the discrepancy when a discrepancy is identified between the first detection image and the first expected image and/or the second detection image and the second expected image.

In some embodiments, the feedback data is configured as a feedback table, wherein the feedback table presents specific discrepancies and the positions of reaction droplets related to the discrepancies.

In some embodiments, the writing control device is configured to control the printing process of the print head based on the feedback data, so that the print head performs supplementary printing on the substrate.

In some embodiments, the dispensing unit includes a pre-processing module, which is configured to pre-process the substrate.

In some embodiments, the pre-processing module is configured to perform hydrophilic treatment on the predetermined reaction sites of the substrate and/or hydrophobic treatment on areas of the substrate other than the predetermined reaction sites.

Through the following detailed description of exemplary embodiments of the present disclosure with reference to the accompanying drawings, other features and advantages of the present disclosure will become clearer.

### BRIEF DESCRIPTION OF THE DRAWINGS

The accompanying drawings, which constitute a part of the specification, illustrate embodiments of the disclosure, and together with the specification, serve to explain principles of the disclosure.

The present disclosure may be more clearly understood from the following detailed description with reference to the accompanying drawings, in which:
FIG. 1 shows a schematic block diagram of a writing system for molecular data storage according to some embodiments of the present disclosure.
Figure 2 shows a schematic diagram of the writing system according to some embodiments of the present disclosure.
Figure 3 shows a schematic diagram of the writing system according to other embodiments of the present disclosure.
Figure 4 shows a schematic diagram of the writing process for molecular data storage, where the print head operates in a scanning manner.
Figure 5 shows a schematic diagram of the writing process for molecular data storage, where the print head operates in a single-pass manner.
Figure 6 shows the arrangement scheme of the print heads in the dispensing unit according to some embodiments of the present disclosure.
Figure 7 shows three exemplary printing methods performed at the reaction sites on the substrate.
Figure 8 shows a block diagram illustrating the principle of degeneracy calculation.
Figure 9 shows an exemplary implementation of degeneracy calculation.
Figure 10 shows another exemplary implementation of degeneracy calculation.
Figure 11 shows a view of a reaction droplet and an oil droplet containing the reaction droplet.
Figure 12 shows a schematic diagram of the reflective effect exhibited by the reaction droplet.
Figure 13 shows a block diagram of the dispensing detection module according to some embodiments of the present disclosure.
Figure 14 shows a schematic diagram of a preprocessed substrate.

Note that in implementations described below, a same reference numeral is sometimes commonly used among different drawings to represent a same part or a part with a same function, and repeated description thereof is omitted. In some instances, similar numbers and letters are used for referring to similar items such that once an item is defined in a drawing, it does not require further discussion in subsequent drawings.

In order to facilitate understanding, positions, dimensions, ranges, and so on of various structures shown in the drawings and the like may sometimes not represent actual positions, dimensions, ranges, and so on. Therefore, the present disclosure is not limited to the positions, dimensions, ranges, and so on disclosed in the drawings and the like.

### DETAILED DESCRIPTION

Various exemplary embodiments of the present disclosure will be described in detail below with reference to the accompanying drawings. It should be noted that relative arrangements of components and steps, numerical expressions, and numerical values set forth in these embodiments do not limit the scope of the disclosure unless otherwise specifically stated.

The following description of at least one exemplary embodiment is merely illustrative in nature and is in no way intended to limit the disclosure, or its application or usage. That is, the structures and methods herein are shown by way of example to illustrate different embodiments of the structures and the methods in the present disclosure. However, those skilled in the art will understand that they are merely illustrative of exemplary ways in which the disclosure may be practiced, instead of exhaustive. Furthermore, the drawings are not necessarily to be drawn in scale, and some features may be exaggerated to illustrate details of particular components.

Techniques, methods and devices known to those of ordinary skill in the relevant art may not be discussed in detail, but where appropriate, such techniques, methods and devices should be regarded as part of the granted specification.

In all examples shown and discussed herein, any specific values are to be construed as illustrative only and not as limitations. Accordingly, other examples of the exemplary embodiments may have different values.

It should be understood that in various exemplary embodiments of the present disclosure, the molecular module may include a deoxyribonucleic acid (DNA), a ribonucleic acid (RNA), a peptide, an organic polymer, an organic small molecule, a carbon nanomaterial, an inorganic substance, and the like. When storing the information, it involves a combination of different molecular modules that represent the content code and the position code. These molecular modules may be combined together through the action mode such as a covalent bond, an ionic bond, a hydrogen bond, an intermolecular force, a hydrophobic force, a complementary base pairing, and the like.

It should be understood that in various exemplary embodiments of the present disclosure, different molecular modules representing different values of the same content code or position code may be of the same type of molecular module, such as all being DNA. Alternatively, different molecular modules representing different values of the same type of content code or position code may be of different types of molecular modules, such as using one type of DNA and one type of RNA to represent two different values of a content code.

Furthermore, molecular modules representing different types of content code or position code may be of the same type of molecular module, such as using different RNAs to represent all content code and position code. Alternatively, different types of molecular modules may be used to represent different types of content code and position code, such as using RNA to represent content code and DNA to represent position code.

Additionally, different molecular modules may be distinguished by at least one of their sequence distribution, sequence length, and secondary structure. For example, different sequences, lengths, or secondary structures of DNA, RNA, peptides, or organic polymers may be used to represent different values of different codes. Furthermore, different chemical forms, physical properties, crystalline or amorphous properties, and morphological characteristics of DNA, RNA, peptides, organic polymers, organic small molecules, carbon nanomaterials, and inorganic substances may also be used to represent different values of different codes.

Moreover, the composition obtained by combining the molecular modules may be a mixture or a compound. For example, the composition may include a plurality of different DNA strands, which may represent one or more initial information fragments in the initial information respectively, and the DNA strands may be mixed together to represent the complete initial information. Alternatively, DNA strands representing various initial information fragments may be further synthesized into the longer DNA strand to represent the complete initial information in the form of compound.

When combining molecular modules representing various codes in sequence, molecular modules representing position code may be combined in front of, behind, or inserted within molecular modules representing the corresponding content code, with no limitation imposed herein. Additionally, molecular modules representing a certain position code or content code may include multiple molecular fragments, which may also be deployed at intervals. For example, molecular modules representing second position encoded data may be combined in front of, behind, or inserted in the middle of the molecular modules representing the respective second content encoded data.

Furthermore, in order to enable different molecular modules to be connected to each other sequentially, in some embodiments, when selecting a molecular module, the molecular module with a respective terminal portion may be selected, that is, the molecular fragment as the terminal portion is a part of the respective molecular module to achieve the sequential connection of the different molecular modules.

In other embodiments, combining the determined molecular modules in order, such that the composition corresponds to the initial information may include: forming the terminal portions corresponding to a preset order at the connection ends of the determined molecular modules; and mixing the molecular modules formed with the respective terminal portions to produce the composition corresponding to the initial information. That is, the molecular fragment serving as the terminal portion is added to the respective molecular module after the molecular module is determined.

For example, during the combination process, different terminal portions may be added to the tail of the first molecular module to enable it to connect with the second, third, and fourth molecular modules, respectively, to form the correct molecular chain/strand.

In a specific example, DNA single strands or double strands may be used as molecular modules. DNA consists of bases, deoxyribose, and phosphate, wherein the bases include four types in total: adenine (A), guanine (G), thymine (T) and cytosine (C). Accordingly, the terminal portions of DNA molecular modules may include sticky ends.

When assembling the DNAs representing different codes, a ligase may be used for sequentially combining the determined multiple DNA strands corresponding to the respective position code and content code. Alternatively, a linker provided at the end of the DNA strand may be used for sequentially combining the determined multiple DNA strands under the action of a ligase. Alternatively, a polymerase chain reaction (PCR) may be used for sequentially combining the determined DNAs. In some PCR methods, the determined multiple DNA strands are used as templates, and the DNA strands containing respective sequences at the connecting ends of two segments of DNA strands to be connected are amplified. In some PCR methods, the DNA strands to be connected may be amplified directly.

In another specific example, RNAs may be used as the molecular modules. RNA is composed of phosphate, ribose and bases. The bases of RNAs mainly include four types: A (adenine), G (guanine), C (cytosine), and U (uracil). Accordingly, a terminal portion of an RNA molecular module may include corresponding functional groups.

A linker sequence provided at the end of the RNA strand and a linker sequence of the DNA may be used for sequentially combining the determined multiple RNA strands under the action of a ligase.

In yet another specific example, peptides may be used as the molecular modules. An amino group of one amino acid can condense with a carboxyl group of another amino acid to form a peptide, and the resulting amide group is referred to a peptide bond in protein chemistry. Molecules of amino acids are the smallest and those of proteins are the largest. Two or more amino acids are dehydrated and condensed to form several peptide bonds to form one peptide chain. Multiple peptide chains undergo multi-level folding to form one protein molecule. Proteins are sometimes referred to "polypeptides". Accordingly, terminal portions of a peptide molecular module may include corresponding functional groups. The determined peptides can be connected under the action of a catalyst to represent at least part of the initial information.

Additionally, as described above, different secondary structures of molecular modules may be used to represent different values of different codes. Molecular modules with different secondary structures may be connected together under the action of ligases.

Next, with reference to the accompanying drawings, some aspects of the present disclosure will be exemplarily introduced.

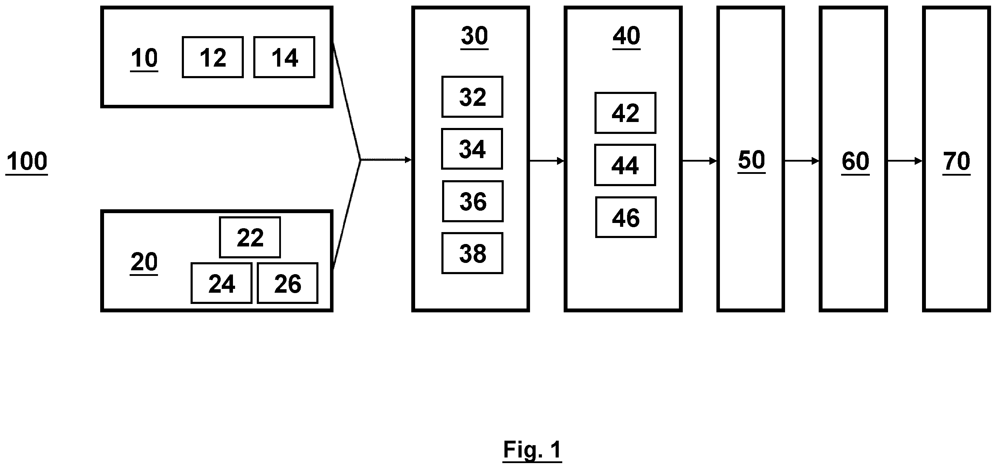

Referring to Figures 1 to 5, schematic diagrams of a writing system 100 for molecular data storage and its writing process according to some embodiments of the present disclosure are shown. The writing system 100 may include an encoded data parsing unit 10, a storage unit 20, a dispensing unit 30, a reaction unit 40, and some subsequent processing units 50, 60, 70 (which will be described in detail later), and the like.

The encoded data parsing unit 10 may include a molecular data encoding system 12 and a molecular data code translation system 14. The molecular data encoding system 12 may be configured to acquire initial information to be stored and convert the initial information to be stored into encoded molecular module assembly data. It should be understood that the encoded data parsing unit 10 may also only include the molecular data code translation system, which may be configured to directly receive the encoded molecular module assembly data.

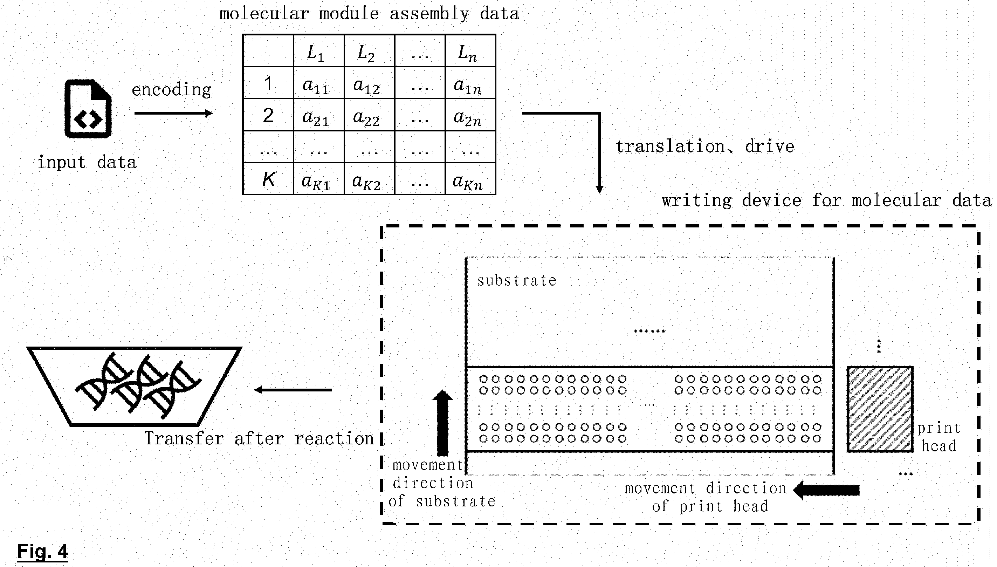

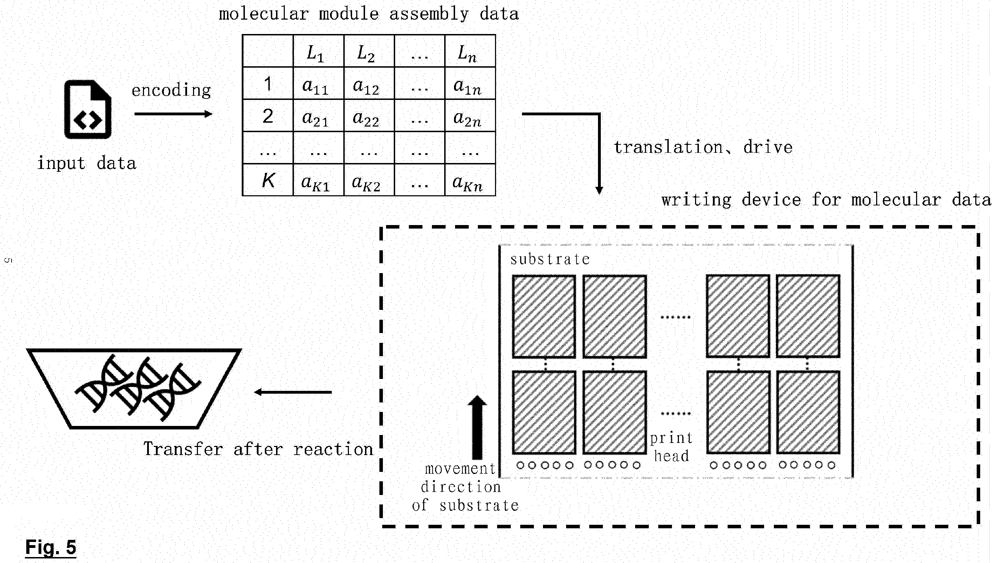

As shown in Figures 4 and 5, the initial information to be stored may be electronic data in any form, such as binary data in the form of "0, 1" as input data. The initial information to be stored may also be electronic data in any other base, such as octal, decimal, hexadecimal, etc.

It should be understood that, in addition to (discrete) digital information, other continuous information, such as analog signals, may be converted into digital information according to quantization methods in specific fields, and further stored using the methods described in the present disclosure.

The molecular module assembly data may include position encoded data and content encoded data associated with the position encoded data. That is, the initial information to be stored may be represented by position encoded data and content encoded data, wherein the position information in the initial information may be represented by corresponding position encoded data, and the content information at each position of the initial information may be represented by corresponding content encoded data.

In some embodiments, the molecular data encoding system 12 may be configured to: acquire the initial information to be stored and represent the initial information using first position encoded data and first content encoded data, wherein each position in the initial information is represented by first position encoded data corresponding one-to-one to that position, and the content information at each position of the initial information is represented by corresponding first content encoded data; recode each first position encoded data to represent the corresponding first position encoded data using first recoded information with a first preset bit number and a first preset base; determine corresponding molecular modules based on the first content encoded data and the first recoded information; and sequentially combine the determined molecular modules so that the composition corresponds to the initial information.

In some embodiments, the molecular data encoding system 12 may be configured to: encode the content information in the initial information into content encoded data, wherein the content encoded data comprises content information encoded data and feature information encoded data associated with the content information.

In some embodiments, the molecular data encoding system 12 may be configured to: determine different molecular modules for the first content encoded data and the first recoded information, respectively.

In some embodiments, the molecular data encoding system 12 may be configured to: determine different molecular modules for contents on different bits in the first recoded information respectively, and determine different molecular modules for different contents on a same bit in the first recoded information respectively.

In some embodiments, the molecular data encoding system 12 may be configured to: for each first recoded information, represent the first recoded information using second position encoded data and second content encoded data, wherein each position in the first recoded information is represented by second position encoded data corresponding one-to-one to that position, and the content at each position in the first recoded information is represented by corresponding second content encoded data; and determine corresponding molecular modules based on the first content encoded data, second position encoded data, and second content encoded data.

In some embodiments, the molecular data encoding system 12 may be configured to: determine different molecular modules for the first content encoded data, second position encoded data, and second content encoded data, respectively.

In some embodiments, the molecular data encoding system 12 may be configured to: determine different molecular modules for the first content encoded data with different values, respectively; or determine different molecular modules for the second position encoded data with different values, respectively; or determine different molecular modules for the second content encoded data with different values, respectively.

In some embodiments, the molecular data encoding system 12 may be configured to: determine different molecular modules for combinations of two codes with different values from the first content encoded data, second position encoded data, and second content encoded data, respectively.

In some embodiments, the molecular data encoding system 12 may be configured to: determine different molecular modules for combinations of the second position encoded data and the second content encoded data with different values, respectively.

The molecular data code translation system 14 may be configured to acquire the encoded molecular module assembly data and parse the molecular module assembly data into writing process control instructions. The writing process control instructions may be understood as machine instructions for controlling the operation of the writing process.

In some embodiments, the writing process control instructions may include molecular module scheduling instructions for the storage unit 20, and the molecular module scheduling instructions may be used to schedule the storage unit 20 to provide reaction droplets containing the required molecular modules.

In some embodiments, the writing process control instructions may include molecular module dispensing instructions for the dispensing unit 30, and the molecular module dispensing instructions may be used to control the dispensing unit 30 to perform quantitative and positional dispensing of reaction droplets.

In some embodiments, the molecular data code translation system 14 may be configured to: acquire molecular module assembly data in the form of a first matrix, wherein assembly information associated with predetermined reaction sites on the substrate is stored in the first matrix.

In some embodiments, the molecular module assembly data may be described as a first matrix of m*n, wherein any element doti-j in the first matrix may represent a corresponding composition of the substance to be dispensed. That is, any element doti-j in the first matrix may represent a corresponding combination of molecular modules.

Regarding the first matrix, refer to Table 1. As shown in Table 1, the element doti-j may represent the composition of the substance(s) in the form of a vector or a vector group. For example, the element doti-j may represent the corresponding composition of the X dispensed substances in the form of a vector (m1, m2, m3, ..., mX).

In some embodiments, the composition of the substance(s) represented by the corresponding vector may include, but is not limited to: one or more molecular modules, one or more enzymes, and/or one or more additives. It should be understood that not all elements in the first matrix need to be assigned corresponding vectors. It is possible that only a portion of the elements in the first matrix are assigned corresponding vectors.

**Table 1: the first matrix**

| | | | | | | | |
|---|---|---|---|---|---|---|---|
| dot1-1 | dot1-2 | dot1-3 | dot1-4 | dot1-5 | dot1-6 | ... ... | dot1-n |
| dot2-1 | dot2-2 | dot2-3 | dot2-4 | dot2-5 | dot2-6 | ... ... | dot2-n |
| dot3-1 | dot3-2 | dot3-3 | dot3-4 | dot3-5 | dot3-6 | ... ... | dot3-n |
| ... ... | ... ... | ... ... | ... ... | ... ... | ... ... | ... ... | ... ... |
| dotm-1 | dotm-2 | dotm-3 | dotm-4 | dotm-5 | dotm-6 | ... ... | dotm-n |

In some embodiments, the molecular data code translation system 14 may be configured to: convert the first matrix into a second matrix, wherein operation instructions for molecular module dispensing are stored in the second matrix.

In some embodiments, the molecular data code translation system 14 may be configured to: convert the first matrix into multiple second matrices associated with multiple print heads 32 in the dispensing unit 30, wherein operation instructions for the corresponding print head 32 are stored in each second matrix.

In some embodiments, the first matrix may be converted into multiple m*n second matrices, and each second matrix may, for example, represent the distribution of at least one substance.

Regarding the second matrix, refer to Table 2. As shown in Table 2, the distribution of substance K is illustrated. For any element Mkx-y in the second matrix, it may have two states (0 or 1), namely, the states of dispensing and not dispensing. Therefore, the information within the element Mkx-y may be represented by 1 bit (0 or 1). In some embodiments, multiple bits may be used to represent the information within an element Mkx-y, to indicate additional information related to requirements such as splicing, calculation, transmission, and the like.

**Table 2: the second matrix**

| | | | | | | | |
|---|---|---|---|---|---|---|---|
| Mk1-1 | Mk1-2 | Mk1-3 | Mk1-4 | Mk1-5 | Mk1-6 | ... ... | Mk1-n |
| Mk2-1 | Mk2-2 | Mk2-3 | Mk2-4 | Mk2-5 | Mk2-6 | ... ... | Mk2-n |
| Mk3-1 | Mk3-2 | Mk3-3 | Mk3-4 | Mk3-5 | Mk3-6 | ... ... | Mk3-n |
| ... ... | ... ... | ... ... | ... ... | ... ... | ... ... | ... ... | ... ... |
| Mkm-1 | Mkm-2 | Mkm-3 | Mkm-4 | Mkm-5 | Mkm-6 | ... ... | Mkm-n |

In some embodiments, the molecular data code translation system 14 may be configured to: combine the operation instructions stored in the second matrix into writing process control instructions.

Taking the second matrix in Table 2 as an example, starting from element Mk1-1, every predetermined number (e.g., 8) of elements may be concatenated into a predetermined (e.g., 1) byte until all elements in the second matrix are concatenated, forming a data block of m*n/8 bytes. To form the writing process control instructions, an instruction file including a file header and a data area may be defined, and the corresponding data block may be loaded into this instruction file.

Continuing with reference to Figures 1 to 5, the storage unit 20 of the writing system 100 is introduced. The storage unit 20 may be configured to provide the reaction substances required for the dispensing unit 30.

The storage unit 20 of the writing system 100 may include multiple storage containers 22 for various predetermined molecular modules. In some embodiments, a solution and molecular modules dissolved in the solution may be stored in the respective storage container 22. The solution may also be referred to as a "base solution," which may include water and basic substances required for the reaction, such as salts.

In some embodiments, the storage unit 20 may include storage containers 22 for storing enzymes involved in the reaction process.

In some embodiments, additives may be stored in the corresponding storage containers 22, and the additives may be configured to promote the reaction process in which the corresponding molecular modules participate and/or facilitate the dispensing process of the corresponding molecular modules by the dispensing unit 30. The additives include salts, glycerol, polyethylene glycol, and/or surfactants. In some embodiments, the additives may be configured to accelerate the reaction process in which the corresponding molecular modules participate. In some embodiments, the additives may be configured to optimize the dispensing process of the corresponding molecular modules by the dispensing unit 30, for example, to achieve the viscosity, surface tension, and other parameters required for maintaining the ideal working state of a specific print head 32, ensuring consistency, stability, and/or smoothness of each print head 32 during the dispensing process.

In some embodiments, the storage unit 20 may include a molecular module supply device 24. The molecular module supply device 24 may connect the corresponding storage containers 22 to the dispensing unit 30 via multiple supply pipelines, and to deliver the corresponding molecular modules and/or enzymes (e.g., liquids containing the corresponding molecular modules and/or enzymes) to the dispensing unit 30 via the corresponding supply pipelines using driving forces such as gravity or negative pressure, according to the writing process control instructions.

In some embodiments, the storage unit 20 may include a molecular module preparation device 26, which may be configured to synthesize predetermined molecular modules and/or replicate predetermined molecular modules. For example, the molecular module preparation device 26 may be configured to synthesize DNA molecular modules in real time based on pre-designed DNA molecular module base sequences. For example, the molecular module preparation device 26 may be configured to replicate DNA molecular modules based on existing DNA molecular modules, thereby achieving the preparation process of DNA molecular modules to provide raw materials for DNA molecular module supply and subsequent processes.

Continuing with reference to Figures 1 to 5, the dispensing unit 30 of the writing system 100 is introduced next. The dispensing unit 30 may be a reactive molecular module quantitative dispensing reaction system, also known as a molecular data writing device, which can achieve the positional and quantitative dispensing of different types of molecular modules and reaction systems on a substrate. In some embodiments, the dispensing unit 30 may advantageously achieve automated, ultra-high-throughput, ultra-micro, and/or rapid positional and quantitative dispensing of molecular modules. In some embodiments, the volume of the reaction droplet involved in each dispensing of molecular modules may be at the nanoliter (nL) or picoliter (pL) level, i.e., each dispensing involves several nanoliters or several picoliters of reaction droplet volume.

In some embodiments, the dispensing unit 30 may be configured to acquire corresponding molecular modules from the storage unit 20 and dispense the corresponding molecular modules to predetermined reaction sites on the substrate according to the writing process control instructions, wherein each of the predetermined reaction sites is provided with molecular modules required for one or more predetermined reaction processes.

To implement the printing process, the dispensing unit 30 may include a print head 32 and a writing control device 34 for controlling the printing process of the print head 32. The writing control device 34 may be configured to control the relative displacement between the print head 32 and the substrate according to the writing process control instructions, so that the print head 32 dispenses reaction droplets containing the corresponding molecular modules to the predetermined reaction sites on the substrate in accordance with the molecular module assembly data. The writing control device 34 may be understood as one or more controllers, which may include at least one memory storing the writing process control instructions; and at least one processor configured to execute the writing process control instructions, wherein the at least one processor implements the printing process of the print head 32 when executing the writing process control instructions.

In some embodiments, the print head 32 may include a piezoelectric print head, a thermal bubble print head, or a solenoid valve print head. In some embodiments, the corresponding print head 32 may quantitatively generate reaction droplets containing specific molecular modules at a specified frequency (e.g., 30 kHz or 10 kHz) and print the corresponding reaction droplets to the predetermined reaction sites on the substrate. Advantageously, reaction droplets containing different molecular modules may achieve micron-scale (µm) co-location printing on demand.

In some embodiments, referring to Figure 4, the print head 32 may be configured to dispense reaction droplets containing the corresponding molecular modules to the predetermined reaction sites on the substrate in a scanning printing manner.

When scanning printing is used, the array of reaction droplets on the substrate may be generated sequentially by a single print head 32 or in parallel by multiple print heads 32 working collaboratively.

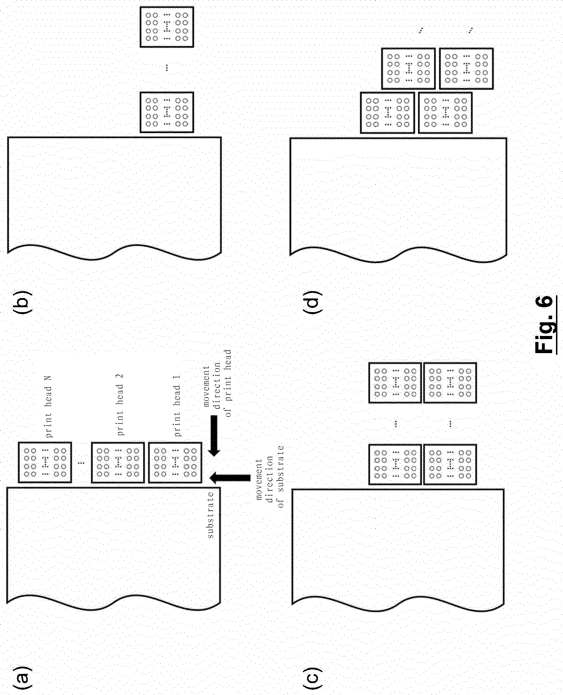

When operating in a scanning printing mode, the print heads 32 of the dispensing unit 30 may form various arrangements. In some embodiments, the print heads 32 may form a single-column arrangement parallel to the movement direction of the substrate (refer to Figure 6a). In some embodiments, the print heads 32 may form a single-row arrangement parallel to the movement direction of the print heads 32 (refer to Figure 6b). In some embodiments, the print heads 32 may form a matrix arrangement (refer to Figure 6c). In some embodiments, the print heads 32 may form an irregular arrangement (refer to Figure 6d).

In some embodiments, each print head 32 may include multiple nozzles, and the multiple nozzles may be configured to dispense reaction droplets containing predetermined molecular modules at nanoliter (nL) or picoliter (pL) level to the predetermined reaction sites on the substrate respectively. In some embodiments, different nozzles of each print head 32 may print reaction droplets containing the same or different substances, such as molecular modules, according to the needs of the actual application.

In some embodiments, referring to Figure 5, the print head 32 may be configured to dispense reaction droplets containing the corresponding molecular modules to the predetermined reaction sites on the substrate in a single-pass printing manner. When single-pass printing is used, the array of reaction droplets on the substrate may be generated by sequentially passing the substrate through the corresponding print heads 32, thereby achieving an efficient printing process.

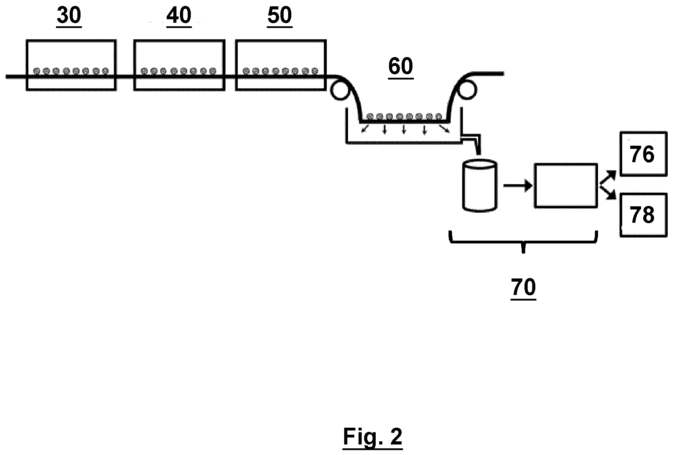

In some embodiments, referring to Figure 2, the substrate may be configured as a continuous substrate and transported in a roll-to-roll manner. The substrate may pass through multiple rollers to achieve the desired tension.

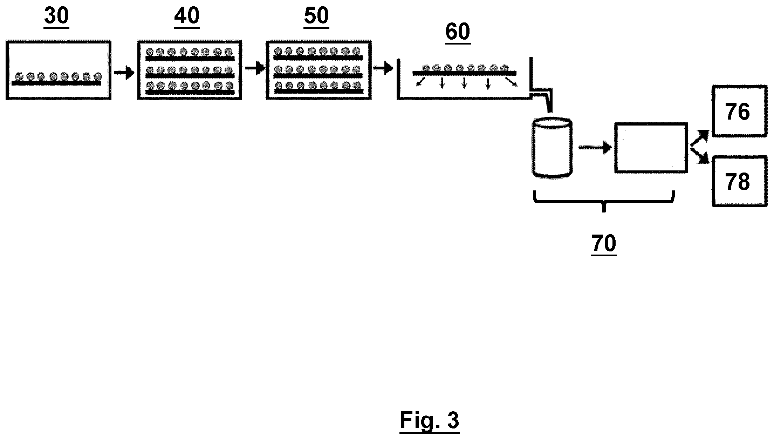

In some embodiments, referring to Figure 3, the substrate may be configured as a discrete substrate and transported in a sheet-to-sheet manner.

In some embodiments, the dispensing unit 30 may include a static electricity eliminator 36 for eliminating static electricity from the substrate. In some embodiments, the static electricity eliminator 36 may be configured as an ion flow electrostatic eliminator, a radioactive isotope electrostatic eliminator, or a sparkless electrostatic eliminator. Thus, when the substrate enters the dispensing unit 30, any static electricity on the substrate may be eliminated, effectively preventing static electricity from negatively affecting the substrate or the reactions occurring on the substrate.

In some embodiments, the dispensing unit 30 may include a cleaning liquid chamber and cleaning pipelines, wherein the cleaning liquid chamber stores cleaning liquid for cleaning the print head 32 and the associated cleaning pipelines. For example, the print head 32 may be connected to at least two isolated chambers, with the first chamber storing reaction droplets and the second chamber storing cleaning liquid. The cleaning liquid in the cleaning liquid chamber may be used to clean the associated cleaning pipelines and the print head 32 (its multiple nozzles) via a valve device. It should be understood that the cleaning liquid in the cleaning liquid chamber does not contain any substances involved in the reaction.

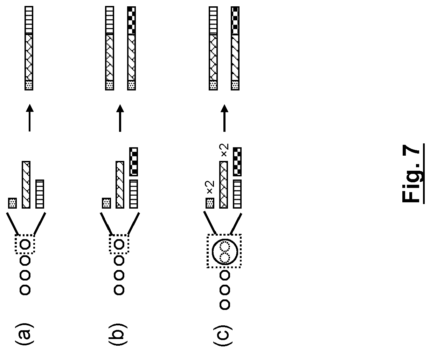

Next, referring to Figures 7 to 10, the printing scheme on the substrate executed by the dispensing unit 30 is described in detail.

The reaction droplet(s) at an individual reaction site on the substrate may function as one or more reaction units 40, wherein the reaction droplet(s) are provided with the molecular modules required for one or more predetermined reaction processes, thereby generating one or more predetermined compositions or effective molecular products at the reaction site. It should be understood that the morphology of the reaction droplet units is controllable through different generation methods of printing reaction droplets. The array of reaction droplets at different sites on the substrate may be arranged in any form.

As shown in Figure 7a, the reaction droplet(s) at an individual reaction site on the substrate may function as a single reaction unit 40, wherein the reaction droplet(s) are provided with the molecular modules required for a single predetermined reaction process, thereby generating a single predetermined composition or effective molecular product at the reaction site.

To improve writing efficiency, as shown in Figure 7b, the reaction droplet(s) at an individual reaction site on the substrate may function as multiple reaction units 40, wherein the reaction droplets are provided with the molecular modules required for multiple predetermined reaction processes, thereby generating multiple predetermined compositions or effective molecular products at the reaction site. To ensure the sufficiency and stability of the reaction process at the reaction site, as shown in Figure 7c, the volume of reaction droplets containing different molecular modules may be adjusted at the corresponding reaction site on the substrate. For example, the redundancy of specific molecular module/s may be increased through multiple printings by the print head 32.

To this end, the molecular module assembly data may be configured as degeneracy-calculated molecular module assembly data, such that the content encoded data for at least a portion of the predetermined reaction sites involves molecular modules required for multiple predetermined reaction processes, thereby generating corresponding multiple predetermined compositions at the at least a portion of the predetermined reaction sites on the substrate respectively.

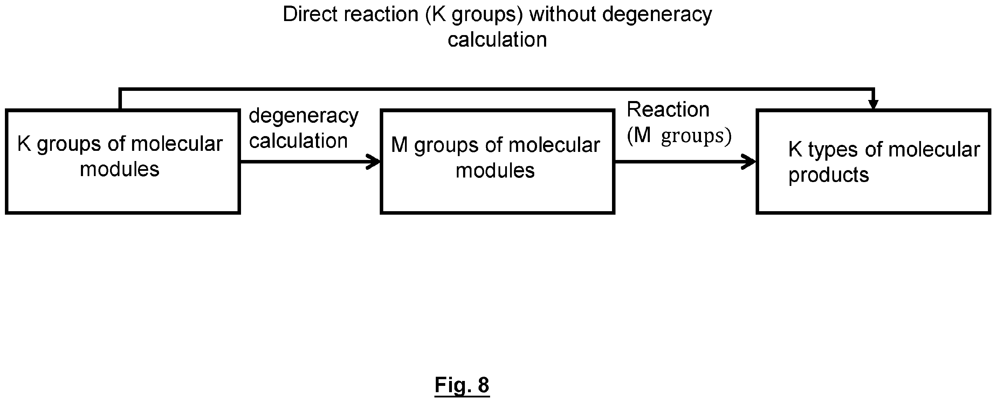

In the specific implementation process, after the molecular module assembly data (as shown in Table 1 above) is determined, an optimized combination of molecular modules may be formed by combining multiple combinations of molecular modules (i.e., so-called degeneracy calculation). Figure 8 illustrates the optimization process of molecular module assembly data, wherein K groups of molecular modules may be optimized into M groups of molecular modules (M ≤ K) through degeneracy calculation, and the reaction of the M groups of molecular modules may yield the corresponding K predetermined compositions, thereby achieving the effects of reducing the number of reactions, accelerating reaction efficiency, and reducing the space occupied by the reactions.

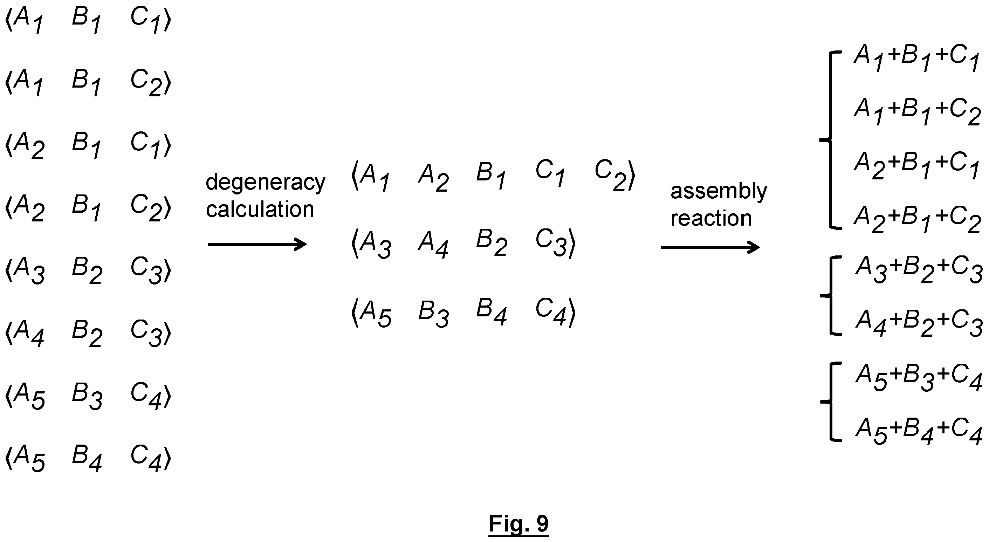

For example, Figure 9 illustrates a specific example of degeneracy calculation. In the initial molecular module assembly data, eight groups of molecular modules (each of which may exemplarily include three molecular modules) may participate in reactions at eight reaction sites, respectively, to generate eight predetermined compositions. After degeneracy calculation, the eight groups of molecular modules may be optimized into three groups of molecular modules (each of which may exemplarily include more than three molecular modules, such as five molecular modules, four molecular modules, and four molecular modules, respectively). These three groups of molecular modules may participate in reactions at three reaction sites, respectively, to generate the eight predetermined compositions.

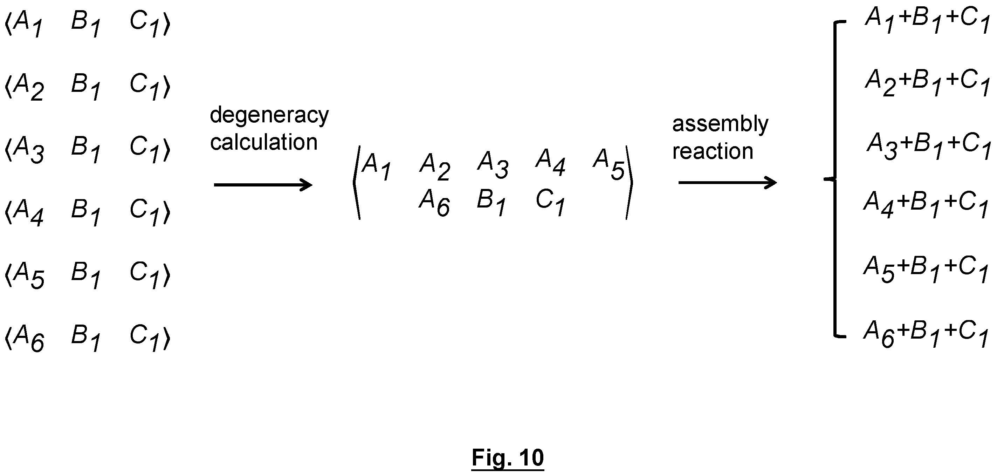

For example, Figure 10 illustrates another specific example of degeneracy calculation. In the initial molecular module assembly data, six groups of molecular modules (each of which may exemplarily include three molecular modules) may participate in reactions at six reaction sites, respectively, to generate six predetermined compositions. After degeneracy calculation, the six groups of molecular modules may be optimized into one group of molecular modules (this group may exemplarily include eight molecular modules), and this group of molecular modules may participate in a reaction at one reaction site to generate the six predetermined compositions.

Based on degeneracy calculation, larger volumes of reaction droplets may be provided at the corresponding reaction sites, leading to one or more of the following benefits: First, the surface area occupied by the combination of the molecular modules on the substrate increases, which improves the tolerance for the printing accuracy of the dispensing unit 30; Second, due to the increased tolerance for printing accuracy, the print heads 32 of the dispensing unit 30 can move at higher speeds in the scanning direction, thereby improving overall printing efficiency; Third, the volume of reaction droplets at an individual reaction site increases (in the example of Figure 10, for instance, from 3 reaction droplet volumes to 8 reaction droplet volumes), making the reaction droplets less prone to evaporation and thereby enhancing the reaction effectiveness.

To achieve an optimized printing process, the writing control device 34 may be configured to control the printing process of the print head 32 according to the writing process control instructions, such that molecular modules required for a first number of predetermined reaction processes are dispensed to a first portion of predetermined reaction sites on the substrate, thereby generating corresponding first number of predetermined compositions at the first portion of predetermined reaction sites respectively, and molecular modules required for a second number of predetermined reaction processes are dispensed to a second portion of predetermined reaction sites on the substrate, thereby generating corresponding second number of predetermined compositions at the second portion of predetermined reaction sites respectively, wherein the first number is greater than the second number.

In some embodiments, the writing control device 34 may be configured to control the printing process of the print head 32 according to the writing process control instructions, such that molecular modules required for multiple predetermined reaction processes are dispensed to a first portion of predetermined reaction sites on the substrate, thereby generating corresponding multiple predetermined compositions at the first portion of predetermined reaction sites respectively, and molecular modules required for only one predetermined reaction process are dispensed to a second portion of predetermined reaction sites on the substrate, thereby generating corresponding only one predetermined composition at the second portion of predetermined reaction sites respectively.

In some embodiments, compared to the second portion of predetermined reaction sites, the first portion of predetermined reaction sites occupies a larger area on the substrate respectively. In some embodiments, compared to the second portion of predetermined reaction sites, the first portion of predetermined reaction sites is dispensed with more types of molecular modules respectively. In some embodiments, compared to the second portion of predetermined reaction sites, the first portion of predetermined reaction sites is dispensed with larger volumes of reaction droplets respectively.

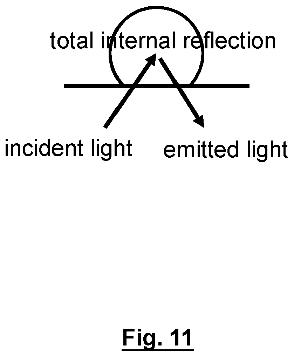

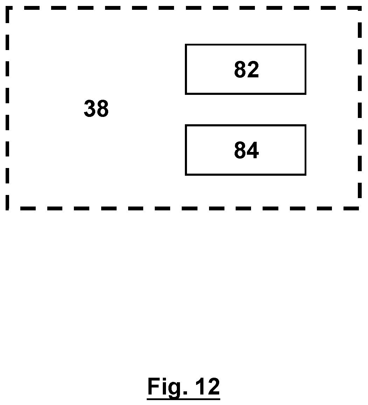

Next, referring to Figures 11 and 12, the dispensing detection module 38 in the dispensing unit 30 is described in detail. To evaluate the dispensing accuracy of the dispensing unit 30, the dispensing unit 30 may include a dispensing detection module 38. The dispensing detection module 38 may be configured to detect the accuracy of the dispensing of reaction droplets by the print head 32.

In some embodiments, the evaluation by the dispensing detection module 38 may be performed in real time. In some embodiments, the evaluation by the dispensing detection module 38 may also be performed at predetermined intervals, for example, before each printing session. In some embodiments, the evaluation by the dispensing detection module 38 may be triggered manually, for example, by pressing a specific button. In some embodiments, the evaluation by the dispensing detection module 38 may be performed automatically. In some embodiments, the evaluation by the dispensing detection module 38 may be performed manually.

In some embodiments, the detection of reaction droplet dispensing may be performed using a visual sensor. In some embodiments, the dispensing detection module 38 may include a microscope device, which may allow operators to observe directly.

In some embodiments, the dispensing detection module 38 may include an image acquisition device 82, such as a camera. Since the reaction droplet is a micron-sized reaction droplet, when incident light passes through the hydrophobic medium from the back of the substrate and enters the corresponding reaction droplet, total internal reflection occurs within the reaction droplet (refer to Figure 11). The image acquisition device 82 may capture images of at least a part of the substrate from the back of the substrate to acquire corresponding detection image (hereinafter referred to as a first detection image), which may reflect the distribution of reaction droplets on the substrate. In some embodiments, the image acquisition device 82 and/or a light source may be installed on the back of the substrate, enabling the image acquisition device 82 to acquire the first detection image of at least a part of the substrate.

Continuing with reference to Figure 12, the dispensing detection module 38 may include an analysis device 84, such as a controller, which is connected to the image acquisition device 82. In some embodiments, the analysis device 84 may be integrated into the writing control device 34 as a functional unit within the writing control device 34. In some embodiments, the analysis device 84 may be a separate functional unit.

The analysis device 84 may store a first expected image related to the at least a part of the substrate. The first expected image may be generated based on the writing process control instructions or molecular module assembly data. The analysis device 84 may be configured to compare the first detection image with the first expected image. In some embodiments, the analysis device 84 may be configured to compare the first detection image with the first expected image in terms of the positional distribution of reaction droplets, the size of reaction droplets, and/or the color of reaction droplets in the image. When the analysis device 84 identifies discrepancy between the first detection image and the first expected image, it may generate feedback data to reflect the discrepancy and/or reflect possible causes of faults or the print heads 32 involved in the faults. The feedback may take various forms. In some embodiments, the analysis device 84 may generate feedback data to prompt a display device to show the existence of discrepancy, thereby drawing the attention of operators. In some embodiments, the feedback data may be configured as a feedback table, which may present specific discrepancy and the positions of the reaction droplets involved in the discrepancy. Accordingly, the writing control device 34 may be configured to control the printing process of the print head 32 based on the feedback data, enabling the print head 32 to perform supplementary printing on the substrate, thereby ensuring that the dispensing results on the substrate conform to the molecular module assembly data.

Additionally or alternatively, the dispensing unit 30 may include a test area, and the writing control device 34 may be configured to control the printing process of the print head 32 according to test instructions, enabling the print head 32 to dispense reaction droplets containing predetermined molecular modules to predetermined test sites in the test area. In some embodiments, the image acquisition device 82 may be configured to acquire a second detection image of the test area, which may reflect the distribution of reaction droplets in the test area.

The analysis device 84 may store a second expected image related to the test area. The analysis device 84 may be configured to compare the second detection image with the second expected image. In some embodiments, the analysis device 84 may be configured to compare the second detection image with the second expected image in terms of the positional distribution of reaction droplets, the size of reaction droplets, and/or the color of reaction droplets in the image. When the analysis device 84 identifies discrepancy between the second detection image and the second expected image, it may generate feedback data to reflect the discrepancy and/or possible causes of faults or the print heads 32 involved in the faults. The feedback may take various forms. In some embodiments, the analysis device 84 may generate feedback data to prompt a display device to show the existence of discrepancy, thereby drawing the attention of operators. In some embodiments, the feedback data may be configured as a feedback table, which may present specific discrepancy and the positions of the reaction droplets involved in the discrepancy. Accordingly, the writing control device 34 may be configured to control the printing process of the print head 32 based on the feedback data, enabling the print head 32 to perform supplementary printing on the substrate, thereby ensuring that the dispensing results on the substrate conform to the molecular module assembly data.

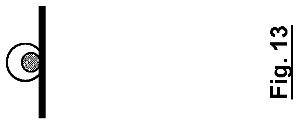

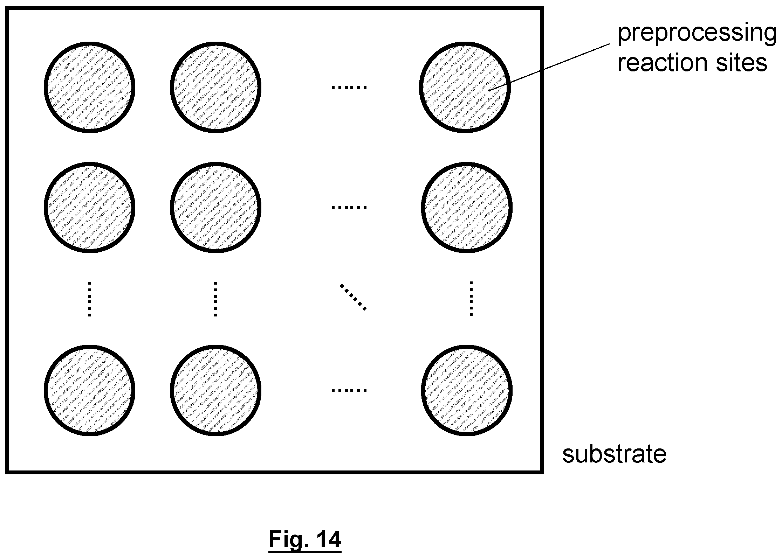

Next, referring to Figures 13 and 14, the preprocessing module in the dispensing unit 30 is described in detail. The dispensing unit 30 may include a preprocessing module, which may be configured to preprocess the substrate to optimize the dispensing and/or reaction process of reaction droplets on the substrate. In some embodiments, the preprocessing module may be configured to: perform hydrophilic treatment on the predetermined reaction sites of the substrate, and/or perform hydrophobic treatment on areas of the substrate other than the predetermined reaction sites, to prevent erroneous mixing of adjacent reaction droplets. In some embodiments, the hydrophilic or hydrophobic treatment may involve physical coating. In some embodiments, the hydrophilic or hydrophobic treatment may also involve chemical modification, where hydrophilic or hydrophobic molecules are covalently bonded to the surface of the substrate. Hydrophilic molecules may include, for example, polyvinyl alcohol, silanes with hydrophilic groups, and the like. Hydrophobic molecules may include, for example, silanes with hydrophobic groups, and the like. In some embodiments, multiple preprocessing reaction sites of different areas (which may have any shape) may be set on the substrate to accommodate reaction units 40 of different volumes, thereby improving the area utilization of the substrate.

It should be understood that the execution of various functional units in the dispensing unit 30 may be controlled by the writing control device 34 according to the present disclosure. The writing control device 34 may include at least one memory storing writing process control instructions; and at least one processor configured to execute the writing process control instructions, wherein the at least one processor implements the execution steps according to some embodiments of the present disclosure when executing the writing process control instructions.

The processor may perform various actions and processing according to instructions stored in the memory. Specifically, the processor may be an integrated circuit chip with signal processing capabilities. The processor may be a general-purpose processor, a digital signal processor (DSP), an application-specific integrated circuit (ASIC), a field-programmable gate array (FPGA), or other programmable logic devices, discrete gate or transistor logic devices, or discrete hardware components.

The memory stores executable instructions that, when executed by the processor, implement the encoding method described above. The memory may be volatile memory or non-volatile memory, or may include both volatile and non-volatile memory. Non-volatile memory may include read-only memory (ROM), programmable read-only memory (PROM), erasable programmable read-only memory (EPROM), electrically erasable programmable read-only memory (EEPROM), or flash memory. Volatile memory may include random access memory (RAM), which serves as external cache memory. By way of example but not limitation, many forms of RAM are available, such as static random access memory (SRAM), dynamic random access memory (DRAM), synchronous dynamic random access memory (SDRAM), double data rate synchronous dynamic random access memory (DDR SDRAM), enhanced synchronous dynamic random access memory (ESDRAM), synchlink dynamic random access memory (SLDRAM), and direct rambus random access memory (DR RAM).

Next, referring to Figures 2 and 3, a series of devices downstream of the dispensing unit 30 in the writing system 100 are introduced, such as the reaction unit 40, the reaction termination unit 50, the elution unit, the reaction droplet collection unit 70, and the like.

As shown in Figures 2 and 3, the reaction unit 40 may be a temperature- and/or humidity-controlled chamber to facilitate DNA assembly reactions in the reaction droplets. In some embodiments, segmented humidification may be performed, for example, in a first stage, humidification may be performed in an open environment, where the humidification area may only target the printed substrate; in a second stage, the substrate may enter the control chamber to allow the reaction to complete.

As shown in Figure 2, in the roll-to-roll substrate transport mode, multiple rollers may be arranged in the control chamber for the substrate to pass through in a rotating manner, extending the movement distance of the substrate and increasing the time the substrate spends in the control chamber, allowing the reaction to proceed fully.

As shown in Figure 3, in the sheet-to-sheet substrate transport mode, the control chamber may include multiple flat racks for temporarily storing the substrate for a period of time, allowing the reaction to proceed fully.

In some embodiments, the reaction unit 40 may include a physical oscillation module 42, which may be configured to optimize the mixing of reaction droplets at the predetermined reaction sites on the substrate. The mixing of reaction droplets may involve thorough mixing of reaction droplets of different forms or reaction droplets sequentially overlaid at the same site. In practice, the physical oscillation module 42 may also accelerate the mixing of reaction droplets.

In some embodiments, the reaction unit 40 may include an enzyme addition device 44, such as a print head 32, for adding enzymes to the predetermined reaction sites on the substrate. It should be understood that the addition of enzymes may be completed in the dispensing unit 30 or in the reaction unit 40.

In some embodiments, the reaction unit 40 may include an oil droplet addition device 46, such as a print head 32, for adding oil droplets to the predetermined reaction sites on the substrate, so that the oil droplets surround the reaction droplets, thereby reducing the evaporation of the reaction droplets. To this end, oil droplets immiscible with the reaction droplets may first be printed on the substrate, followed by the dispensing of reaction droplets.

Additionally or alternatively, the reaction unit 40 may be configured to adjust and/or optimize the configuration of molecular modules involved in the reaction process (e.g., DNA molecular module base sequences), the component ratios in reaction reagents (e.g., solutions or enzyme solutions), reaction condition parameters, reaction procedures, and other factors, so that more complete reactions can be achieved at lower concentrations on the substrate sites in a shorter time, achieving faster reaction rates and/or saving reaction consumption.

Continuing with reference to Figures 2 and 3, the writing system 100 may include a reaction termination unit 50 and a reaction droplet elution unit 60. The reaction termination unit 50 may be configured to terminate the reactions at the predetermined reaction sites on the substrate. The reaction droplet elution unit 60 may be configured to elute the reaction droplets containing compositions from the substrate into an aqueous or oil phase medium.

In some embodiments, the reaction termination unit 50 may include a temperature adjustment device and/or a humidity adjustment device, which may be configured to deactivate enzymes, for example, by heat deactivation.

In some embodiments, the reaction termination unit 50 may include a termination chemical addition device, which is configured to add termination chemicals to the substrate. The termination chemicals may include ethylenediaminetetraacetic acid (EDTA) and/or salts above a predetermined concentration, and the like.

After the reaction is terminated, the reaction droplet elution unit 60 may be responsible for eluting the reaction droplets from the substrate into an aqueous medium. For example, the reaction droplet elution unit 60 may rinse the substrate with water at normal or high pressure, causing the reacted reaction droplets to be immersed in water.

In some embodiments, the reaction droplet elution unit 60 may be configured to elute reaction droplets by methods such as shaking in an aqueous phase, and/or by using air pressure or surface wiping to remove residual water droplets. Alternatively, the reaction droplet elution unit 60 may be configured to elute the reaction droplets into an oil phase medium.

In some embodiments, reaction termination may be combined with reaction elution. For example, high-temperature water elution or elution with a solvent or solution containing termination chemicals may be used.

Continuing with reference to Figures 2 and 3, the writing system 100 may include a reaction droplet collection unit 70, which may be configured to receive the reaction droplets containing compositions eluted by the reaction droplet elution unit 60.

In some embodiments, the reaction droplet collection unit 70 may include a composition processing module, which is configured to: screen effective compositions from the eluted reaction droplets; and back up the effective compositions through a predetermined amplification method. The predetermined amplification method may include, but is not limited to: PCR, isothermal amplification, thereby achieving efficient expansion of effective compositions, i.e., molecular products.

In some embodiments, the composition processing module is configured to: perform enrichment and collection processing on the eluted reaction droplets before screening.

In some embodiments, the enrichment and collection processing is performed as follows: removing the aqueous phase from the eluted reaction droplets by column or membrane filtration; and collecting effective compositions by elution, magnetic bead methods, oil-water separation, freeze-drying, and/or heat drying.

Continuing with reference to Figures 2 and 3, the writing system 100 may include a molecular data transfer module, which is configured to transfer the collection of effective compositions to a molecular data storage device. The molecular data transfer module may, for example, be responsible for physically transferring molecular sets storing effective data information to specific locations in molecular data storage devices 76, 78 for storage. The storage method may be in the form of solid-state storage 76 or solution storage 78, and the like.

It should be noted that the block diagrams in the drawings illustrate possible implemented architectures, functions and operations of systems according to various embodiments of the present disclosure. In this regard, each block in the block diagrams may represent a module, a program segment, or a portion of code that contains one or more executable instructions for implementing the specified logic functions. It should also be noted that, in some alternative implementations, functions labeled in the blocks may also occur in a sequence different from that labeled in the drawings. For example, two blocks shown one after another may actually execute substantially in parallel, or they may sometimes execute in reverse order, depending on the functionality involved. It will also be noted that each block of the block diagrams, and combinations of blocks in the block diagrams, can be implemented by special purpose hardware-based systems that perform the specified functions or operations, or can be implemented using a combination of specialized hardware and computer instructions.

Generally speaking, the various example embodiments of the present disclosure may be implemented in hardware or special purpose circuits, software, firmware, logic, or any combination thereof. Some aspects may be implemented in hardware, while other aspects may be implemented in firmware or software that may be executed by a controller, microprocessor, or other computing device. While aspects of embodiments of the present disclosure are illustrated or described as block diagrams, flowcharts, or using some other graphical representations, it will be understood that the blocks, apparatus, systems, techniques, or methods described herein may be, by way of non-limiting examples, implemented in hardware, software, firmware, special purpose circuit or logic, general purpose hardware or controllers, or other computing devices, or some combination thereof.

In the description and claims, the words "front", "back", "top", "bottom", "above", "below", etc., if present, are used for descriptive purposes and are not necessarily used for describing a constant relative position. It should be understood that the words so used are interchangeable under appropriate circumstances such that the embodiments of the disclosure described herein, for example, can be operated in other orientations than those illustrated or otherwise described herein.

As used herein, the word "exemplary" means "serving as an example, instance, or illustration" rather than as a "model" that will be reproduced precisely. Any implementation exemplarily described herein is not necessarily to be construed as preferred or advantageous over other implementations. Furthermore, the disclosure is not constrained by any expressed or implied theory presented in the above technical field, background, summary or detailed description.

As used herein, the word "substantially" is meant to include any minor variations resulting from design or manufacturing defects, device or element tolerances, environmental effects, and/or other factors. The word "substantially" also allows for differences from perfect or ideal conditions due to parasitic effects, noise, and other practical considerations that may be present in actual implementations.

Additionally, the foregoing description may refer to elements or nodes or features being "connected "or "coupled "together. As used herein, unless expressly stated otherwise, "connected" means that one element/node/feature is electrically, mechanically, logically, or otherwise directly connected to another element/node/feature (or direct communication). Similarly, unless expressly stated otherwise, "coupled" means that one element/node/feature can be directly or indirectly connected mechanically, electrically, logically, or otherwise to another element/node/feature to allow interactions even though these two features may not be connected directly. That is, "coupled" is intended to encompass both direct connections and indirect connections of elements or other features, including connections utilizing one or more intervening elements.

In addition, "first", "second" and similar terms may also be used herein for reference purposes only and are therefore not intended to be limiting. For example, the words "first", "second", and other such numerical words involving structures or elements do not imply a sequence or order unless expressly stated in the context.

It should also be understood that the words "comprising/including" when used herein illustrate the presence of the indicated features, integers, steps, operations, units and/or components, but does not exclude the presence or addition of one or more other features, integers, steps, operations, units and/or components and/or combinations thereof.

Although some specific embodiments of the present disclosure have been described in detail by way of examples, those skilled in the art will understand that the above examples are for illustration only and are not intended to limit the scope of the disclosure. The various embodiments disclosed herein may be combined in any manner without departing from the spirit and scope of the disclosure. Those skilled in the art will further understand that various modifications may be made to the embodiments without departing from the scope and spirit of the disclosure. The scope of the disclosure is defined by the appended claims.

## Claims

1. A writing system for molecular data storage, **characterized in that** the writing system comprises:
an encoded data parsing unit configured to acquire encoded molecular module assembly data and parse the molecular module assembly data into writing process control instructions;
a storage unit configured to store predetermined types of molecular modules and provide corresponding molecular modules according to the writing process control instructions;
a dispensing unit configured to acquire corresponding molecular modules from the storage unit and dispense the corresponding molecular modules to predetermined reaction sites on a substrate according to the writing process control instructions, wherein each of the predetermined reaction sites is provided with molecular modules required for one or more predetermined reaction processes; and
a reaction unit configured to facilitate one or more predetermined reaction processes at each of the predetermined reaction sites on the substrate respectively, thereby generating one or more predetermined compositions at each of the predetermined reaction sites on the substrate respectively.

2. The writing system according to claim 1, **characterized in that**:
the encoded data parsing unit comprises an encoding module configured to acquire initial information for molecular data storage and encode the initial information into the molecular module assembly data; and/or
the molecular module assembly data comprises position encoded data and content encoded data associated with the position encoded data; and/or
the molecular module assembly data is configured as degeneracy-calculated molecular module assembly data, such that the respective content encoded data for at least a portion of the predetermined reaction sites involves molecular modules required for multiple predetermined reaction processes, thereby generating corresponding multiple predetermined compositions at the at least a portion of the predetermined reaction sites on the substrate respectively.

3. The writing system according to claim 1, **characterized in that** the encoded data parsing unit comprises a translation module configured to acquire molecular module assembly data and convert the molecular module assembly data into writing process control instructions, wherein the writing process control instructions comprise molecular module scheduling instructions for the storage unit and molecular module dispensing instructions for the dispensing unit.

4. The writing system according to claim 1, **characterized in that** the dispensing unit comprises a print head and a writing control device for controlling the printing process of the print head, wherein the writing control device is configured to control the relative displacement between the print head and the substrate according to the writing process control instructions, so that the print head dispenses reaction droplets containing corresponding molecular modules to the predetermined reaction sites on the substrate in accordance with the molecular module assembly data.

5. The writing system according to claim 4, **characterized in that**:
the print head is configured to dispense reaction droplets containing corresponding molecular modules to the predetermined reaction sites on the substrate in a scanning printing or single-pass printing manner; and/or
each print head comprises multiple nozzles configured to dispense reaction droplets containing predetermined molecular modules at nanoliter or picoliter level to the predetermined reaction sites on the substrate; and/or
the writing control device is configured to control the printing process of the print head according to the writing process control instructions, such that molecular modules required for a first number of predetermined reaction processes are dispensed to a first portion of the predetermined reaction sites on the substrate respectively, thereby generating corresponding first number of predetermined compositions at the first portion of the predetermined reaction sites respectively, and molecular modules required for a second number of predetermined reaction processes are dispensed to a second portion of the predetermined reaction sites on the substrate respectively, thereby generating corresponding second number of predetermined compositions at the second portion of the predetermined reaction sites respectively, wherein the first number is greater than the second number.

6. The writing system according to claim 5, **characterized in that**:
each of the first portion of the predetermined reaction sites occupies a larger area on the substrate compared to each of the second portion of the predetermined reaction sites; and/or
each of the first portion of the predetermined reaction sites is dispensed more types of molecular modules compared to each of the second portion of the predetermined reaction sites; and/or
each of the first portion of the predetermined reaction sites is dispensed reaction droplets of larger volume compared to each of the second portion of the predetermined reaction sites.

7. The writing system according to claim 4, **characterized in that** the dispensing unit comprises a dispensing detection module configured to detect the accuracy of the dispensing of reaction droplets by the print head.

8. The writing system according to claim 7, **characterized in that**:
the dispensing detection module comprises a microscope device and/or an image acquisition device; and/or
the image acquisition device is configured to acquire a first detection image of at least a part of the substrate; and/or
the dispensing unit comprises a test area, and the writing control device is configured to control the printing process of the print head according to a test instruction, so that the print head dispenses reaction droplets containing predetermined molecular modules to predetermined test sites on the test area; and/or
the image acquisition device is configured to acquire a second detection image of the test area.

9. The writing system according to claim 8, **characterized in that**:
the dispensing detection module comprises an analysis device, wherein a first expected image related to the at least a part of the substrate and/or a second expected image related to the test area is stored in the analysis device, and the analysis device is configured to compare the first detection image with the first expected image and/or the second detection image with the second expected image; and/or
the analysis device is configured to compare the first detection image with the first expected image and/or the second detection image with the second expected image in terms of the positional distribution of reaction droplets, the size of reaction droplets, and/or the color of reaction droplets in the image; and/or
the analysis device is configured to generate feedback data to reflect the discrepancy when a discrepancy is identified between the first detection image and the first expected image and/or the second detection image and the second expected image; and/or
the feedback data is configured as a feedback table, wherein the feedback table presents specific discrepancies and the positions of reaction droplets related to the discrepancies; and/or
the writing control device is configured to control the printing process of the print head based on the feedback data, so that the print head performs supplementary printing on the substrate.

10. The writing system according to claim 1**, characterized in that** the dispensing unit comprises a pre-processing module configured to pre-process the substrate.

11. The writing system according to claim 10, **characterized in that** the pre-processing module is configured to perform hydrophilic treatment on the predetermined reaction sites of the substrate and/or hydrophobic treatment on areas other than the predetermined reaction sites of the substrate.

12. The writing system according to claim 1, **characterized in that**:
the reaction unit comprises a physical oscillation module configured to optimize the mixing of reaction droplets at the respective predetermined reaction sites on the substrate; and/or
the reaction unit comprises an oil droplet addition device for adding oil droplets to the predetermined reaction sites on the substrate, so that the oil droplets surround the reaction droplets respectively, thereby reducing the evaporation of the reaction droplets; and/or
the reaction unit comprises a constant temperature and humidity control chamber, wherein multiple rollers are provided in the control chamber for the substrate to pass through in a rotating manner; and/or
the writing system comprises a reaction termination unit for terminating the reaction at the predetermined reaction sites on the substrate,
wherein the reaction termination unit comprises:
a temperature adjustment device and/or a humidity adjustment device configured to deactivate enzymes; and/or
a termination chemical addition device configured to add termination chemicals to the substrate.

13. The writing system according to claim 3, **characterized in that**:
the content encoded data of the molecular module assembly data comprises content information encoded data and feature information encoded data associated with the content information; and/or
the encoded data parsing unit is configured to acquire molecular module assembly data in the form of a first matrix, wherein assembly information associated with the predetermined reaction sites on the substrate is stored in the first matrix; and/or
the encoded data parsing unit is configured to convert the first matrix into a second matrix, wherein operation instructions for molecular module dispensing are stored in the second matrix; and/or
the encoded data parsing unit is configured to convert the first matrix into multiple second matrices associated with multiple print heads in the dispensing unit; and/or
operation instructions for the corresponding print head are stored in each second matrix; and/or
the encoded data parsing unit is configured to combine the operation instructions stored in the second matrices into writing process control instructions.

14. A writing method for molecular data storage, comprising:
acquiring writing process control instructions converted from encoded molecular module assembly data, wherein the molecular module assembly data comprises position encoded data and content encoded data associated with the position encoded data;
dispensing corresponding molecular modules to predetermined reaction sites on a substrate according to the writing process control instructions, wherein each of the predetermined reaction sites is provided with molecular modules required for one or more predetermined reaction processes respectively, thereby generating one or more predetermined compositions at each of the predetermined reaction sites on the substrate respectively.

15. The writing method according to claim 14, **characterized in that**:
the molecular module assembly data is configured as degeneracy-calculated molecular module assembly data, such that the content encoded data for at least a portion of the predetermined reaction sites involves molecular modules required for multiple predetermined reaction processes respectively, thereby generating corresponding multiple predetermined compositions at the at least a portion of the predetermined reaction sites on the substrate respectively; and/or
"dispensing corresponding molecular modules to predetermined reaction sites on a substrate according to the writing process control instructions" comprises:
controlling the printing process of the print head according to the writing process control instructions, so that molecular modules required for a first number of predetermined reaction processes are dispensed to a first portion of the predetermined reaction sites on the substrate respectively, thereby generating corresponding first number of predetermined compositions at the first portion of the predetermined reaction sites respectively, wherein the first number is greater than or equal to two; and/or
controlling the printing process of the print head according to the writing process control instructions, so that molecular modules required for a second number of predetermined reaction processes are dispensed to a second portion of the predetermined reaction sites on the substrate respectively, thereby generating corresponding second number of predetermined compositions at the second portion of the predetermined reaction sites respectively, wherein the second number is less than the first number.

16. The writing method according to claim 15, **characterized in that**, compared to each of the second portion of the predetermined reaction sites:
each of the first portion of the predetermined reaction sites occupies a larger area on the substrate;
each of the first portion of the predetermined reaction sites is dispensed more types of molecular modules; and/or
each of the first portion of the predetermined reaction sites is dispensed reaction droplets of larger volume.

17. The writing method according to claim 15, **characterized in that**:
the writing method comprises detecting the accuracy of the dispensing of reaction droplets by a dispensing detection module; and/or
"detecting the accuracy of the dispensing of reaction droplets by a dispensing detection module" comprises:
acquiring a first detection image of at least a part of the substrate and comparing the first detection image with a first expected image of the at least a part of the substrate; and/or
acquiring a second detection image of a test area and comparing the second detection image with a second expected image of the test area; and/or
the writing method comprises comparing the first detection image with the first expected image and/or the second detection image with the second expected image in terms of the positional distribution of reaction droplets, the size of reaction droplets, and/or the color of reaction droplets in the image; and/or
the writing method comprises generating feedback data to reflect the discrepancy when the discrepancy is identified between the first detection image and the first expected image and/or the second detection image and the second expected image; and/or
the feedback data is configured as a feedback table, wherein the feedback table presents specific discrepancies and the positions of reaction droplets related to the discrepancies; and/or
the writing method comprises supplementarily dispensing molecular modules to the substrate based on the feedback data.

18. A writing control device for molecular data storage, **characterized in that** the writing control device comprises:
at least one memory storing writing process control instructions; and
at least one processor configured to execute the writing process control instructions, wherein the at least one processor implements the method according to any one of claims 14 to 17 when executing the writing process control instructions.

19. An encoded data parsing unit for molecular data storage, **characterized in that** the encoded data parsing unit comprises:
a molecular data encoding system configured to acquire initial information to be stored and convert the initial information to be stored into encoded molecular module assembly data, wherein the molecular module assembly data comprises position encoded data and content encoded data associated with the position encoded data;
a molecular data code translation system configured to acquire the encoded molecular module assembly data and parse the molecular module assembly data into writing process control instructions,
wherein the molecular data code translation system is configured to:
acquire a first matrix formed by various molecular module assembly data, wherein assembly information associated with the predetermined reaction sites on the substrate is stored in the first matrix;
convert the first matrix into a second matrix, wherein operation instructions for molecular module dispensing are stored in the second matrix;
combine the operation instructions stored in the second matrix into writing process control instructions.

20. A dispensing unit for molecular data storage, **characterized in that** the dispensing unit comprises:
a molecular module dispensing system comprising at least one print head;
a molecular module dispensing control system comprising the writing control device according to claim 18, wherein the writing control device is configured to control the printing process of the print head of the molecular module dispensing system on the substrate.
